(19) **Europäisches Patentamt European Patent Office Office européen des brevets**

(11) **EP 1 849 413 B1**

(12) **FASCICULE DE BREVET EUROPEEN**

(45) Date de publication et mention
de la délivrance du brevet:
**26.11.2008 Bulletin 2008/48**

(51) Int Cl.:
*A61B 6/00* *(2006.01)*     *A61B 5/02* *(2006.01)*

(21) Numéro de dépôt: **07290403.0**

(22) Date de dépôt: **03.04.2007**

(54) **Procédé d'imagerie optique par fluorescence de tissus biologiques, notamment pour délimiter des regions d'intérêt des tissus à analyser par tomographie**

Verfahren der optischen Fluoreszenzbildgebung biologischer Gewebe, insbesondere zur Bestimmung von Interessengebieten für die Gewebeanalyse durch Tomographie

Optical imaging method using fluorescence of biological tissue, in particular for delimiting regions of interest in tissue to be analysed by tomography.

(84) Etats contractants désignés:
**DE FR GB IT**

(30) Priorité: **24.04.2006 FR 0603610**

(43) Date de publication de la demande:
**31.10.2007 Bulletin 2007/44**

(73) Titulaire: **COMMISSARIAT A L'ENERGIE ATOMIQUE**
**75015 Paris (FR)**

(72) Inventeurs:
• **Texier-Nogues, Isabelle**
**38000 Grenoble (FR)**

• **Peltie, Philippe**
**38760 Saint Paul de Varces (FR)**
• **Heinrich, Emilie**
**38360 Sassenage (FR)**
• **Blanc, Rolande**
**38360 Noyarey (FR)**

(74) Mandataire: **Bolinches, Michel Jean-Marie et al**
**Cabinet Orès**
**36, rue de St Pétersbourg**
**75008 Paris (FR)**

(56) Documents cités:
**US-A- 4 541 438**     **US-A- 4 852 579**
**US-A- 5 370 119**     **US-A- 5 697 373**
**US-A1- 2003 013 973**

## Description

La présente invention concerne un procédé d'imagerie optique par fluorescence d'au moins un tissu biologique, notamment pour délimiter des régions d'intérêt du ou des tissu(s) à analyser par tomographie. L'invention s'applique plus particulièrement à des tissus *in vivo.*

De manière connue, l'imagerie optique par fluorescence permet de compléter les divers types d'instruments de médecine nucléaire, comme ceux faisant appel aux techniques de tomographie d'émission de positons (« PET » en abrégé en anglais), de gammatomographie (« SPECT » en abrégé en anglais, i.e. tomographie d'émission monophotonique) ou comme les imageurs aux rayons X (e.g. radio-numériques, tomographie par rayons X) et les imageurs IRM (imagerie par résonance magnétique).

L'imagerie optique par fluorescence nécessite l'injection dans un organisme humain ou animal d'un marqueur (e.g. un conjugué anticorps/fluorophore) qui va se fixer de manière spécifique sur une zone d'intérêt pour le biologiste ou le médecin, par exemple sur une tumeur cancéreuse d'un organe. Par la suite, on entend par « fluorophore » toute structure moléculaire ou particulaire apte à émettre de la lumière en réponse à une excitation lumineuse (e.g. fluorophore organique, nanocristal semi-conducteur, boîte quantique, etc.), et l'on entend par « marqueur » le composé injecté comprenant un tel fluorophore.

On peut ainsi détecter des nodules cancéreux via une technique bien moins invasive et destructrice que l'imagerie par rayonnements ionisants (e.g. aux rayons X ou aux traceurs radioactifs). Par ailleurs, les imageurs optiques offrent une bonne résolution millimétrique.

Enfin, il convient de noter que l'appareillage nécessaire pour une telle imagerie optique est d'une relative simplicité, comprenant notamment une diode laser compacte à titre de source d'éclairage, une caméra haute sensibilité à titre de détecteur, des tables de déplacement motorisées, et est d'un coût très nettement inférieur à celui des appareils d'imagerie utilisant des rayonnements ionisants.

Les appareillages d'imagerie par fluorescence les plus simples comportent une source d'éclairage (e.g. de type fibre, laser, lampe à arc, diodes électroluminescentes) et une caméra filtrée (pour éviter la rétrodiffusion de la lumière d'excitation) permettant l'acquisition d'une image de fluorescence ; on parle alors de « FRI » (« Fluorescence Reflectance Imaging » ou imagerie de fluorescence par réflexion). Les photons pénétrant peu dans les tissus (1 mm environ), on ne localise avec cette technique que des marqueurs se trouvant à la superficie de ces derniers (e.g. que des tumeurs marquées superficielles en cancérologie).

Si le marqueur (e.g. la tumeur marquée) est en profondeur (à 1 cm de profondeur par exemple), il est absolument impossible avec une seule acquisition de type « FRI » de le localiser, en raison de la forte diffusion des photons d'excitation et des photons émis par le fluorophore. C'est la raison pour laquelle on déplace la source d'éclairage, de façon à réaliser un « quadrillage » de la zone à analyser et à acquérir autant d'images que de positions de la source d'éclairage. Une reconstruction complexe à partir de toutes les images acquises permet de reconstruire l'image de fluorescence en « 3D » : on parle alors de tomographie optique. Avec un tel dispositif, on peut imager de petits animaux, tels que des rats ou des souris.

Lorsque l'on procède à l'imagerie optique d'un corps entier de petit animal, on peut généralement :

- soit acquérir une image de fluorescence globale à deux dimensions par la technique « FRI » en quelques secondes tout au plus, avec l'inconvénient de n'avoir aucune information en profondeur dans les tissus,
- soit utiliser la technique de tomographie, qui permet d'avoir une image en trois dimensions moyennant, d'une part, une série d'images respectivement prises pour différentes positions source/ détecteur (acquisition de 10 à 15 min. pour un champ d'environ 1 cm$^2$ et un pas de 2 mm) et, d'autre part, des algorithmes de reconstruction de l'image, laquelle reconstruction sera d'autant plus longue que la zone à reconstruire sera plus vaste. La difficulté rencontrée dans la reconstruction « 3D » par tomographie, lorsque l'on utilise un fluorophore quelconque, réside donc dans le manque d'informations sur la zone restreinte où les marqueurs sont susceptibles de se trouver (ces marqueurs sont en général fixés sur l'organe à observer, comme par exemple une tumeur cancéreuse). On est alors obligé de balayer l'ensemble de l'objet à imager - pour une souris la zone totale est de 50 x 75 mm$^2$ - avec un pas de quelques mm pour éviter des temps d'acquisition trop longs, et l'on perd de l'information entre les points d'excitation.

De plus, les algorithmes de reconstruction d'images utilisent le plus souvent comme point de départ une distribution homogène de sources fluorescentes (i.e. un même niveau de fluorescence, nul ou non) dans le corps entier de l'animal. Cela nécessite donc un nombre élevé d'itérations avant de converger vers une distribution fiable des sources fluorescentes, et peut donc provoquer des erreurs.

Le plus souvent, la longueur d'onde d'excitation utilisée est comprise entre 600 et 800 nm et les fluorophores (typiquement des cyanines de dénominations Cy5, Cy7, Alexa 633 ou Alexa 750) émettent entre 700 et 900 nm. Dans cette plage de longueurs d'onde, l'autofluorescence des tissus biologiques est réduite par rapport à la plage bleu-vert (de 400 à 500 nm), mais cette autofluorescence indésirable est néanmoins toujours présente (l'autofluorescence des tissus biologiques est due à la présence de chromophores endogènes, tels que les porphyrines de l'hémoglobine, les protéines fluorescentes, etc.). En outre, avec un fluorophore fixé sur l'organe à détecter, le rapport signal/bruit présente l'inconvénient d'être relativement réduit.

Un inconvénient majeur de ces techniques connues d'imagerie optique par fluorescence réside donc essentiellement, pour la technique « FRI », dans l'absence d'information en profondeur pour la localisation du marqueur dans l'organe à imager et, pour la tomographie, dans la difficulté d'obtention d'informations en profondeur (direction Z) satisfaisantes en plus de celles relatives à la surface de l'organe (directions X et Y).

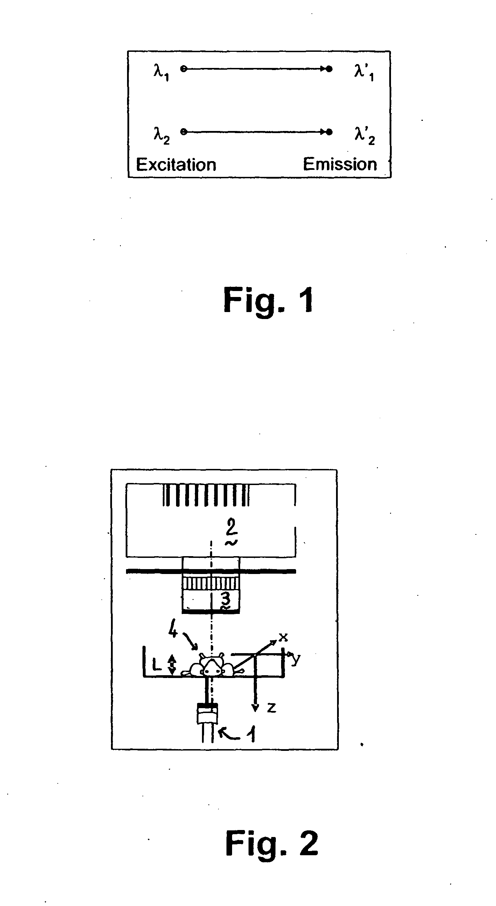

La publication « A dual fluorochrome probe for imaging proteases; M. F. Kircher, R. Weissleder, L. Josephson ; Bioconj. Chem. 2004, 15, 242 » présente une méthode d'imagerie optique consistant à utiliser un marqueur dual formé d'une nanoparticule fonctionnalisée par deux fluorophores organiques (deux cyanines de dénominations Cy5.5 et Cy7), pour évaluer l'activité d'une enzyme dans des tissus. On calcule le ratio des émissions de ces deux fluorophores pour estimer la localisation en profondeur du vecteur marqué dans les tissus. Ce marqueur est soit excité à $\lambda_1$ = 630 nm et analysé à $\lambda'_1$ = 700 nm pour le fluorophore Cy5.5, soit excité à $\lambda_2$ = 736 nm et analysé à $\lambda'_2$ = 800 nm pour le fluorophore Cy7, selon le schéma d'acquisition illustré à la figure 1 jointe, qui montre qu'à chaque longueur d'onde d'excitation $\lambda_1$, $\lambda_2$ correspond une et une seule bande spectrale étroite autour d'un maximum d'émission de fluorescence $\lambda'_1$ ou bien $\lambda'_2$.

Un inconvénient majeur de cette méthode d'imagerie optique à marqueur dual réside dans la complexité de la fabrication de ce marqueur, en raison de l'opération de greffage des deux fluorophores sur un même vecteur.

Un autre inconvénient de cette méthode est que les spectres d'absorption/ d'émission relativement larges de ces deux fluorophores se chevauchent, ce qui engendre des difficultés de filtrage pour détecter séparément chacun des deux fluorophores et pouvoir ainsi obtenir le ratio des deux émissions donnant l'estimation de la profondeur dans les tissus. Par ailleurs, la proximité des deux longueurs d'onde d'absorption et d'émission des deux marqueurs est génératrice de phénomènes parasites, tels que le transfert d'énergie et l'inhibition de la fluorescence.

Un autre inconvénient de cette méthode est que le spectre d'émission de ces deux marqueurs à base de fluorophores organiques peut être décalé, à cause d'interactions avec l'environnement biologique.

Un dernier inconvénient de cette méthode à marqueur dual est que l'utilisation du rapport d'intensité des émissions de fluorescence des deux fluorophores pour estimer la profondeur du marqueur repose sur deux hypothèses contestables, i.e que les coefficients d'absorption respectifs $a_1$ et $a_2$ des tissus aux deux longueurs d'onde d'excitation $\lambda_1$ et $\lambda_2$ sont les mêmes, ainsi que les coefficients d'absorption respectifs $a'_1$ et $a'_2$ des tissus aux deux longueurs d'onde d'émission $\lambda'_1$ et $\lambda'_2$ (soit $a_1=a_2$ et $a'_1=a'_2$). Ces deux hypothèses sont justifiées dans cette publication par la proximité mutuelle des longueurs d'onde $\lambda_1$ et $\lambda_2$, d'une part, et $\lambda'_1$ et $\lambda'_2$, d'autre part. Cependant, il s'avère que cette proximité entre les longueurs d'onde d'excitation et d'émission des deux fluorophores utilisés pénalise le flitrage optique pour la mesure du ratio de fluorescence, ce qui nuit à la qualité de l'évaluation de la profondeur du marqueur dans les tissus.

Le document US-A-5 370 119 présente un procédé pour mesurer le pH d'une cible appropriée permettant d'obtenir des informations d'évolution dans le temps de ce pH (profil cinétique), comprenant :

- la mise en contact de la cible à analyser avec un marqueur fluorescent présentant au moins deux pics d'excitation et un seul pic d'émission et dont le spectre d'émission est dépendant du pH,
- l'excitation de la cible ainsi traitée, successivement à ces longueurs d'onde d'excitation du marqueur fluorescent,
- la mesure de la fluorescence émise par la cible à ces longueurs d'onde d'excitation, et le calcul du pH de cette cible à partir du rapport des signaux de fluorescence émis à partir de ces deux longueurs d'onde d'excitation, par la lecture du pH correspondant au rapport obtenu, sur une courbe d'étalonnage du marqueur, en fonction du pH.

On notera que les marqueurs fluorescents de type à fluorophores organiques, tels que la fluorescéine, qui sont utilisés dans ce document ne sont pas aptes à émettre plusieurs longueurs d'ondes d'émission en réponse à ces longueurs d'onde d'excitation, mais une seule et même longueur d'onde d'émission.

On notera en outre que le procédé de mesure selon ce dernier document ne permet pas une estimation de la localisation tridimensionnelle du marqueur dans le tissu, ni des coefficients d'absorption moyens du tissu vis-à-vis des longueurs d'onde d'excitation.

Un but de la présente invention est de proposer un procédé d'imagerie optique d'au moins un tissu biologique, dans lequel au moins un marqueur fluorescent a été introduit préalablement comprenant :

a) l'excitation dudit ou de chaque marqueur par des rayonnements lumineux incidents et la détection de bandes d'émission relatives à des rayonnements fluorescents émis par ledit ou chaque marqueur en réponse à cette excitation, puis

b) l'analyse des intensités de fluorescence relatives à ces bandes d'émission,

qui permet de remédier aux inconvénients précités.

A cet effet, le procédé selon l'invention est tel que l'étape a) comprend :

- une excitation séquentielle, par $\underline{n}$ longueurs d'onde d'excitation $\lambda_i$ incidentes différentes, dudit ou de chaque marqueur qui est apte à être excité par au moins deux de ces $\underline{n}$ longueurs d'ondes $\lambda_i$ et à émettre en réponse, pour chaque longueur d'onde $\lambda_i$, une série $S_i$ de $\underline{m}$ bandes d'émission $B_j$ simultanées présentant différentes longueurs d'onde maximales $\lambda'_j$ qui sont sensiblement les mêmes d'une série $S_i$ à une autre (où n et m sont indépendamment l'un de l'autre des entiers égaux ou supérieurs à 2, et où i et j varient respectivement de 1 à n et de 1 à m), et
- une détection de ces séries $S_i$ au moins au nombre de deux qui comprennent chacune ces $\underline{m}$ bandes $B_j$ émises simultanément, pour en déduire à l'étape b) une estimation de la localisation tridimensionnelle dudit ou de chaque marqueur dans le ou chaque tissu et/ou des coefficients d'absorption moyens du ou de chaque tissu vis-à-vis des longueurs d'onde d'excitation $\lambda_i$.

Selon une autre caractéristique de l'invention, ledit ou chaque marqueur peut être à base d'un fluorophore ou groupe de fluorophores qui est apte à être excité par ces longueurs d'ondes $\lambda_i$ et à émettre simultanément, en réponse à chacune d'entre elles, ces $\underline{m}$ bandes $B_j$.

On notera que la méthode d'imagerie optique selon l'invention permet de délimiter, en un temps d'acquisition relativement bref du fait de l'estimation de localisation tridimensionnelle obtenue, les zones d'intérêt du ou des tissu(s) à imager de façon plus approfondie par tomographie, en procurant bien plus d'informations sur ce(s) tissu(s) qu'une simple image obtenue par la méthode d'imagerie optique « FRI », notamment grâce à la détection en profondeur dans la direction Z du ou de chaque marqueur dans le(s) tissu(s) qui s'ajoute aux deux seules directions superficielles X et Y accessibles par imagerie « FRI ».

On notera également que cette méthode selon l'invention permet de disposer d'une information sur les coefficients d'absorption moyens du ou de chaque tissu.

Ainsi, cette méthode selon l'invention permet de procurer simplement, grâce à l'utilisation de marqueurs judicieux, un point de départ intéressant pour les algorithmes de reconstruction d'images de corps entiers d'animaux en améliorant la qualité de la reconstruction tout en réduisant sa durée d'acquisition. En effet, contrairement aux algorithmes de reconstruction traditionnels qui utilisent des coefficients d'absorption tabulés et l'hypothèse d'une répartition *a priori* homogène des sources fluorescentes dans chaque animal (choisie généralement la même d'un animal à l'autre), la méthode de l'invention utilise comme point de départ de reconstruction une image qui est propre à chaque animal et contenant déjà des informations tridimensionnelles et des valeurs moyennes des coefficients d'absorption des tissus relatifs à l'animal examiné.

Par « cartographie d'absorption », on entend de manière connue dans la présente description la carte d'atténuation du ou de chaque tissu à la longueur d'onde d'excitation lorsque l'on travaille en transmission (source d'éclairage d'un côté de l'objet à examiner et caméra de détection de l'autre).

Par « cartographie d'émission », on entend de manière connue l'image de fluorescence du ou de chaque tissu lorsqu'il est excité à une longueur d'onde donnée et que l'on recueille le signal à une ou plusieurs autres longueurs d'onde.

Par « cartographie de rétro-diffusion », on entend de manière connue la carte de diffusion du ou de chaque tissu à la longueur d'onde d'excitation (la source d'éclairage et la caméra de détection étant alors placées toutes deux d'un même côté de l'objet à examiner).

Selon une autre caractéristique de l'invention, l'étape b) peut comprendre également une détermination d'un ou de plusieurs ratio(s) d'émission entre les $\underline{m}$ longueurs d'onde maximales $\lambda'_j$, et d'un ou plusieurs ratio(s) de transmission du ou de chaque tissu entre les $\underline{n}$ longueurs d'onde d'excitation $\lambda_i$, pour l'obtention d'une cartographie d'émission du ou de chaque tissu.

Selon un mode de réalisation de l'invention, on utilise un dispositif d'imagerie optique de type fonctionnant en transmission, comportant une source pour lesdits rayonnements incidents et un détecteur qui sont situés respectivement des deux côtés dudit ou de chaque tissu à imager.

Selon une variante de réalisation de l'invention, on utilise un dispositif d'imagerie optique comportant une source pour lesdits rayonnements incidents et un détecteur qui sont situés tous deux d'un même côté dudit ou chaque tissu à imager. Avantageusement, on peut dans ce cas déduire également de l'étape a) précitée une cartographie de rétrodiffusion dudit ou de chaque tissu aux $\underline{n}$ longueurs d'onde d'excitation $\lambda_i$.

De préférence, les $\underline{n}$ longueurs d'onde d'excitation $\lambda_i$ sont décalées deux à deux entre elles d'un intervalle au moins égal à 100 nm et, à titre encore plus préférentiel, au moins égal à 150 nm.

Egalement à titre préférentiel, les $\underline{m}$ longueurs d'onde maximales $\lambda'_j$ desdites bandes d'émission $B_j$ sont décalées deux à deux entre elles d'un intervalle au moins égal à 100 nm.

Avantageusement, les $\underline{n}$ longueurs d'onde d'excitation $\lambda_i$ sont toutes comprises entre 750 nm et 1000 nm.

Egalement avantageusement, les $\underline{m}$ longueurs d'onde maximales $\lambda'_j$ desdites bandes d'émission $B_j$ sont toutes comprises entre 450 nm et 800 nm.

Selon un exemple préférentiel de réalisation de l'invention, l'étape a) précitée comprend :

- l'excitation successive, par deux longueurs d'ondes d'excitation différentes $\lambda_1$ et $\lambda_2$, dudit marqueur qui est apte à être excité par ces deux longueurs d'ondes $\lambda_1$ et $\lambda_2$ et à émettre en réponse sensiblement une même série $S_1$, $S_2$ de deux bandes d'émission $B_1$ et $B_2$ présentant respectivement deux longueurs d'onde maximales différentes $\lambda'_1$ et $\lambda'_2$ ($\underline{n} = \underline{m} = 2$), et

- la détection des deux séries $S_1$ et $S_2$ comprenant chacune ces deux bandes $B_1$ et $B_2$ émises simultanément.

Conformément à cet exemple préférentiel, ledit marqueur est avantageusement à base d'un fluorophore ou groupe de fluorophores qui est apte à être excité par ces deux longueurs d'onde $\lambda_1$ et $\lambda_2$ et à émettre simultanément, en réponse à chacune d'entre elles, les deux bandes $B_1$ et $B_2$.

Selon une autre caractéristique de l'invention, ledit ou chaque marqueur peut avantageusement comprendre un fluorophore à base d'au moins un nanocristal inorganique semi-conducteur de type convertisseur élévateur de fréquence (appelé « up-converting nanocrystal » en anglais).

Avantageusement, ledit ou chaque nanocristal peut comprendre alors au moins :

- un oxyde ou un oxysulfure d'un métal choisi dans le groupe constitué par l'yttrium, le vanadium et les terres rares (i.e. par définition un élément de la classification de Mendeleev de numéro atomique allant de 57 à 71, e.g. le gadolinium), et
- un ion émetteur, tel qu'un cation métallique de terre rare comme le terbium, l'erbium ou l'europium.

L'absorbeur que peut comprendre en outre ledit ou chaque nanocristal peut être lui aussi sous forme d'ion, par exemple un ion d'ytterbium.

Encore plus avantageusement, ledit ou chaque nanocristal peut être à base d'un oxyde d'yttrium répondant à la formule $Y_2O_3$: $Er^{3+}$, $Yb^{3+}$, où Er et Yb sont respectivement l'erbium et l'ytterbium et sont chacun présents dans ledit ou chaque nanocristal selon un taux de dopage allant de 1 % à 20 %.

On notera que les marqueurs usuels, qu'ils soient à base de fluorophores organiques ou à base de nanocristaux inorganiques semi-conducteurs (i.e. « quantum dots » en anglais) qui sont au contraire de type convertisseur abaisseur de fréquence (« down-converting nanocrystals » en anglais), sont connus pour n'émettre qu'à une seule longueur d'onde et ne sont donc pas utilisables dans la méthode de la présente invention.

On notera également que ces nanocristaux convertisseurs élévateurs de fréquence absorbent des photons de faible énergie (typiquement dans le domaine infrarouge) et émettent des photons d'énergie plus élevée (typiquement dans le domaine visible), ce qui rend ces nanocristaux très intéressants comme marqueurs pour l'imagerie optique du petit animal *in vivo.* En effet, ces nanocristaux sont résistants au photo-blanchiement, émettent des longueurs d'ondes auxquelles les phénomènes d'autofluorescence sont minimisés ou évités (du fait que ces processus indésirables ont toujours lieu à une longueur d'onde plus élevée que la longueur d'onde d'excitation), et présentent des spectres d'émission à bandes très étroites.

On notera toutefois que de nouveaux fluorophores autres que ces nanocristaux inorganiques de type convertisseur élévateur de fréquence pourraient être utilisables dans la méthode selon l'invention, pourvu qu'ils soient aptes à être excités par au moins deux longueurs d'onde d'excitation différentes $\lambda_i$ et à émettre simultanément en réponse à chacune d'entre elles au moins deux longueurs d'ondes d'émission différentes $\lambda'_i$.

Avantageusement, ledit ou chaque tissu imagé dans la méthode de l'invention est de type *in vivo,* ledit ou chaque marqueur étant à base d'un conjugué fluorophore/ ligand biologique, tel qu'un conjugué nanocristal inorganique fonctionnalisé de type convertisseur élévateur de fréquence / biomolécule. Cette dernière peut être un peptide, un oligonucléotide, de l'ADN, une protéine, etc.

On peut par exemple utiliser pour l'obtention de ces nanocristaux fonctionnalisés la méthode de fonctionnalisation décrite dans le document FR-A-2 812 662 au nom de la Demanderesse, qui présente l'utilisation d'un silane-époxyde pour l'obtention d'une fonction diol qui peut ensuite être activée en aldéhyde pour réagir avec des fonctions amines de biomolécules.

On notera que les marqueurs utilisables dans la méthode selon l'invention peuvent présenter, outre les propriétés optiques précitées, d'autres propriétés utiles en imagerie. Ils peuvent notamment être également en eux-mêmes, ou être liés à:

- des agents de contraste pour l'IRM, tels que par exemple des chélates de gadolinium ou des nanoparticules d'oxydes de fer ou de gadolinium (ils peuvent également comporter un coeur d'oxyde de fer ou de gadolinium), et à
- des agents de contraste pour l'imagerie « PET », « SPECT », l'imagerie aux rayons X ou pour tout autre type d'imagerie.

Ainsi, ledit ou chaque marqueur de l'invention peut comprendre en outre au moins un élément, par exemple choisi dans le groupe constitué par les chélates de gadolinium, les nanoparticules d'oxydes de fer et les nanoparticules de gadolinium, qui est apte à faire dudit marqueur un agent de contraste présentant une autre propriété non optique utilisable par exemple dans les techniques d'imagerie précitées.

On notera en outre que les marqueurs utilisables dans la méthode selon l'invention peuvent être constitués d'une seule entité présentant les propriétés optiques voulues, ou bien d'un ensemble de telles entités assemblées en une nanostructure, de façon à constituer une bibliothèque de marqueurs différents pouvant être détectés simultanément ou séparément.

Comme indiqué précédemment, on peut utiliser une cartographie d'émission et/ou d'absorption et/ou de rétrodiffusion spécifiquement obtenue(s) à l'étape b) pour ledit ou chaque tissu, pour délimiter au moins une région d'intérêt dudit ou de chaque tissu à analyser par tomographie, et l'on utilise avantageusement cette délimitation de ladite ou chaque région d'intérêt propre audit ou à chaque tissu comme point de départ pour une reconstruction d'images par tomographie.

Encore plus avantageusement, ladite ou lesdites cartographie(s) sont relatives à un corps entier d'animal.

Les caractéristiques précitées de la présente invention, ainsi que d'autres, seront mieux comprises à la lecture de la description suivante de plusieurs exemples de réalisation de l'invention, donnés à titre illustratif et non limitatif, ladite description étant réalisée en relation avec les dessins joints, parmi lesquels :

la figure 1 est un diagramme illustrant de façon symbolique un principe d'acquisition d'image selon un état antérieur de la technique relatif à une méthode d'imagerie optique à marqueur dual,

la figure 2 est une vue schématique de profil d'une installation de tomographie configurée en transmission, qui est utilisable pour mettre en oeuvre la méthode d'imagerie optique selon l'invention,

la figure 3 est un diagramme illustrant de façon symbolique le principe d'acquisition d'image selon un exemple préférentiel de mise en oeuvre de la méthode d'imagerie optique selon l'invention,

la figure 4 est un graphique illustrant de manière schématique l'analyse spectrale obtenue par la méthode de l'invention symbolisée à la figure 3,

la figure 5 est un schéma illustrant les éléments essentiels d'un banc de caractérisation optique utilisé en relation avec des fluorophores selon l'invention de type nanocristaux convertisseurs élévateurs de fréquence,

la figure 6 est un graphique illustrant les deux bandes d'émission obtenues pour ces nanocristaux à l'état de poudre lors d'une excitation à une première longueur d'onde $\lambda_1$, incluant une vue de détail de la première de ces deux bandes d'émission,

la figure 7 est un graphique illustrant les deux mêmes bandes d'émission obtenues pour ces nanocristaux à l'état de poudre lors d'une excitation à une seconde longueur d'onde $\lambda_2$, incluant également une vue de détail de la première de ces deux bandes d'émission,

la figure 8 contient deux graphiques illustrant respectivement, pour ces deux longueurs d'onde d'excitation $\lambda_1$ et $\lambda_2$, les ratio de fluorescence obtenus pour les deux bandes d'émission des figures 6 et 7 en fonction des taux de dopage des ions utilisés pour ces nanocristaux,

la figure 9 est un graphique illustrant les deux bandes d'émission similaires obtenues pour ces nanocristaux à l'état de poudre ou en solution dans un milieu liquide lors d'une excitation à la première ou seconde longueurs d'onde $\lambda_1$ ou $\lambda_2$, et avec filtre ou sans filtre anti-diffusion, et

la figure 10 présente des images de fluorescence obtenues avec les niveaux de gris correspondants, pour ces mêmes nanocristaux ayant été disposés à différentes profondeurs dans un fantôme d'animal avec capillaire, à l'état de poudre ou à différentes concentrations dans un milieu liquide.

Comme illustré à la figure 2, on a utilisé pour l'ensemble des exemples et essais présentés ci-après un dispositif expérimental de tomographie configuré en transmission, incluant une source d'éclairage 1 laser émettant dans le domaine proche infrarouge et une caméra de détection 2 à haute sensibilité pourvue d'une lentille 3 qui sont respectivement situées en avant et en arrière d'un petit animal 4 à imager (par exemple une souris).

On a notamment utilisé des cartographies d'émission acquises en transmission et des cartographies d'absorption. Ce dispositif pourrait également être tel que la source d'éclairage 1 soit disposée du même coté de l'animal 4 que la caméra 2, et l'on utiliserait dans ce cas les cartographies d'émission et de rétrodiffusion.

## 1) <u>Description détaillée de la méthode selon l'invention :</u>

Sur la figure 2, L désigne l'épaisseur totale de l'animal, supposée connue et mesurée par un autre dispositif, et z la profondeur des fluorophores dans la direction Z de l'animal 4 (X et Y étant les deux autres directions à la surface de l'animal 4). On a mesuré les signaux d'intensités lumineuses $I(\lambda_1, \lambda'_1)$ et $I(\lambda_2, \lambda'_2)$ avec des excitations à $\lambda_1$ et $\lambda_2$ et une détection à $\lambda'_1$ et $\lambda'_2$ tant pour $\lambda_1$ que pour $\lambda_2$.

La transmission de la lumière est donnée de façon connue par $I(\lambda_1) = I_0(\lambda_1) e^{-a_1 z}$ et $I(\lambda_2) = I_0(\lambda_2) e^{-a_2 z}$, où $a_1$ et $a_2$ représentent respectivement les coefficients d'absorption moyens des tissus à $\lambda_1$ et $\lambda_2$. $I_0$ l'intensité incidente et z la distance parcourue dans les tissus. Si l'on ne considère pour simplifier le problème que l'absorption des tissus en négligeant la diffusion, on a 5 inconnues:

- la profondeur du fluorophore en z, et
- les coefficients d'absorption des tissus aux différentes longueurs d'onde: $\lambda_1$, $\lambda_2$, $\lambda'_1$, $\lambda'_2$, notées respectivement $a_1$, $a_2$, $a'_1$, $a'_2$.

Les rendements quantiques de fluorescence des fluorophores sont notés $\eta(\lambda_1, \lambda'_1)$, $\eta(\lambda_1, \lambda'_2)$, $\eta(\lambda_2, \lambda'_1)$ et $\eta(\lambda_2, \lambda'_2)$. Ces rendements quantiques peuvent être mesurés indépendamment au spectrophotomètre.

On a donc besoin de 5 équations pour résoudre le système.

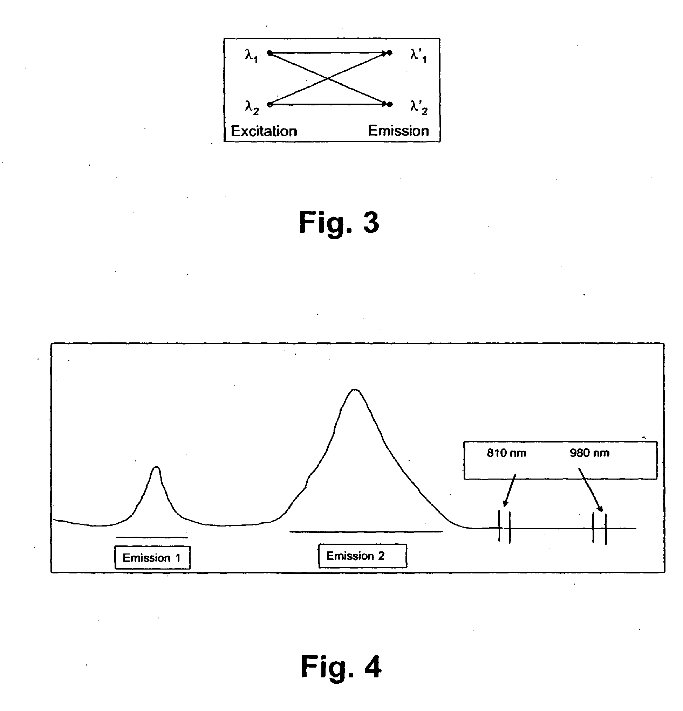

La figure 3 illustre un exemple préférentiel du principe de détection qui caractérise la méthode d'imagerie de l'invention, et selon lequel :

- un fluorophore unique a la propriété d'émettre simultanément à au moins deux longueurs d'onde différentes $\lambda'_1$ et $\lambda'_2$ lorsqu'il est excité à une longueur d'onde $\lambda_1$ ou $\lambda_2$ donnée (obtention d'une cartographie d'émission), et
- ce même fluorophore s'excite à au moins deux longueurs d'onde différentes $\lambda_1$ et $\lambda_2$ (obtention de cartographies de rétrodiffusion et/ou d'absorption).

Par l'acquisition de 4 ou 6 images correspondant à la transmission à $\lambda_1$ et $\lambda_2$, et aux émissions $\lambda_1/(\lambda'_1$ et $\lambda'_2)$, $\lambda_2/(\lambda'_1$ et $\lambda'_2)$ ou $\lambda_1/\lambda'_1$, $\lambda_1/\lambda'_2$, $\lambda_2/\lambda'_1$, $\lambda_2/\lambda'_2$ (ou plus si plus de longueurs d'onde d'excitation et/ou d'émission sont envisagées), on peut rapidement et simplement, d'une part, délimiter les zones d'intérêt à imager de façon plus approfondie par tomographie, par la réalisation d'une cartographie d'absorption, de rétrodiffusion ou d'émission et, d'autre part, fournir comme point de départ pour la reconstruction d'image par tomographie une répartition des sources fluorescentes dans l'animal avec une image propre à chaque animal et incluant déjà des information en X,Y, Z. On améliore ainsi la rapidité et la justesse de cette reconstruction d'image.

Le principe de l'acquisition d'image selon l'invention qui est illustré à la figure 4 consiste essentiellement à :

(i) réaliser une excitation du fluorophore à la première longueur d'onde d'excitation $\lambda_1$ et à enregistrer la transmission à la même longueur d'onde $\lambda_1$,
(ii) enregistrer soit simultanément - si on dispose d'un dispositif d'acquisition avec analyse spectrale, soit successivement, les émissions fluorescentes de ce fluorophore aux deux longueurs d'onde maximales $\lambda'_1$ et $\lambda'_2$, puis
(iii) à mettre à nouveau en oeuvre ces étapes (i) et (ii) avec la seconde longueur d'onde d'excitation $\lambda_2$ appliquée à ce même fluorophore.

Les calculs simplifiés ci-après montrent que l'utilisation selon l'invention de plusieurs longueurs d'onde d'excitation $\lambda_1$ et $\lambda_2$ et d'émission $\lambda'_1$ et $\lambda'_2$ permet d'apporter beaucoup plus d'informations que les méthodes d'imagerie optique connues, telles que la méthode « FRI », non seulement sur une estimation de la profondeur en z des fluorophores mais encore sur les coefficients d'absorption moyens des tissus.

Pour déduire la profondeur z du fluorophore de l'acquisition précitée selon l'invention, on dispose avantageusement de mesures supplémentaires pour le même nombre de 5 inconnues. On a :

- $I(\lambda_1, \lambda'_1) = f(a_1, a'_1)$: mesure n°1 avec excitation à $\lambda_1$ et émission à $\lambda'_1$,

$$I(\lambda_1, \lambda'_1) = I_1^0 \eta(\lambda_1, \lambda'_1) e^{-a_1(L-z)} e^{-a'_1 z}$$

- $I(\lambda_1, \lambda'_2) = f(a_1, a'_2)$: mesure n°2 avec excitation à $\lambda_1$ et émission à $\lambda'_2$,

$$I(\lambda_1, \lambda'_2) = I_1^0 \eta(\lambda_1, \lambda'_2) e^{-a_1(L-z)} e^{-a'_2 z}$$

- $I(\lambda_2, \lambda'_1) = f(a_2, a'_1)$: mesure n°3 avec excitation à $\lambda_2$ et émission à $\lambda'_1$,

$$I(\lambda_2, \lambda'_1) = I_2^0 \eta(\lambda_2, \lambda'_1) e^{-a_2(L-z)} e^{-a'_1 z}$$

- $I(\lambda_2, \lambda'_2) = f(a_2, a'_2)$: mesure n°4 avec excitation à $\lambda_2$ et émission à $\lambda'_2$,

$$I(\lambda_2, \lambda'_2) = I_2^0 \eta(\lambda_2, \lambda'_2) e^{-a_2(L-z)} e^{-a'_2 z}$$

- ratio des transmissions des tissus à $\lambda_1$ et $\lambda_2$: $I(\lambda_1, \lambda'_1)/ I(\lambda_2, \lambda'_2) = f(a_1, a_2)$: mesure n°5

$$\frac{I(\lambda_1, \lambda'_1)}{I(\lambda_2, \lambda'_2)} = \frac{I_1^0}{I_2^0} e^{-(a_1 - a_2)L}$$

Par conséquent, on dispose d'assez d'équations pour résoudre le système, sans hypothèse *a priori* sur les coefficients d'absorption, lesquels peuvent même être déterminés très simplement comme suit.

- Le ratio des transmissions des tissus (mesure n°5) donne la différence des coefficients d'absorption moyens ($a_1-a_2$) aux longueurs d'onde d'excitation, moyennant de connaître les intensités incidentes $I_1^0$ et $I_2^0$ délivrées par les sources d'excitation à $\lambda_1$ et $\lambda_2$, et l'épaisseur de l'animal L, données que l'on peut mesurer expérimentalement.
- Le ratio des émissions mesurées à $\lambda'_1$ (ou à $\lambda'_2$) donne alors la profondeur z du fluorophore par l'équation suivante, moyennant de connaître le ratio des rendements quantiques d'émission à $\lambda'_1$ lors des excitation à $\lambda_1$ et $\lambda_2$, donnée propre au marqueur et pouvant être mesurée indépendamment au spectrofluorimètre:

$$\frac{I(\lambda_1, \lambda'_1)}{I(\lambda_2, \lambda'_1)} = \frac{I_1^0 \eta(\lambda_1, \lambda'_1) e^{-(a_1 - a_2)(L-z)}}{I_2^0 \eta(\lambda_2, \lambda'_1)} = \frac{I(\lambda_1, \lambda_1)}{I(\lambda_2, \lambda_2)} \times \frac{\eta(\lambda_1, \lambda'_1)}{\eta(\lambda_2, \lambda'_1)} \times e^{(a_1 - a_2)z}$$

- Si on le souhaite, on peut mesurer la différence des coefficients d'absorption ($a'_1-a'_2$) aux longueurs d'onde d'émission par le ratio des émissions à $\lambda'_1$ et $\lambda'_2$, lors de l'excitation soit à $\lambda_1$, soit à $\lambda_2$:

$$\frac{I(\lambda_1, \lambda'_1)}{I(\lambda_1, \lambda'_2)} = \frac{\eta(\lambda_1, \lambda'_1)}{\eta(\lambda_1, \lambda'_2)} \times e^{-(a'_1 - a'_2)z}$$

On notera que les calculs ci-dessus sont excessivement simplifiés, car ils ne tiennent pas compte de la diffusion qui est très importante aux longueurs d'onde utilisées, et du fait que plusieurs sources fluorescentes peuvent être présentes.

**2) Essais mettant en évidence les propriétés optiques de fluorophores selon la méthode de l'invention :**

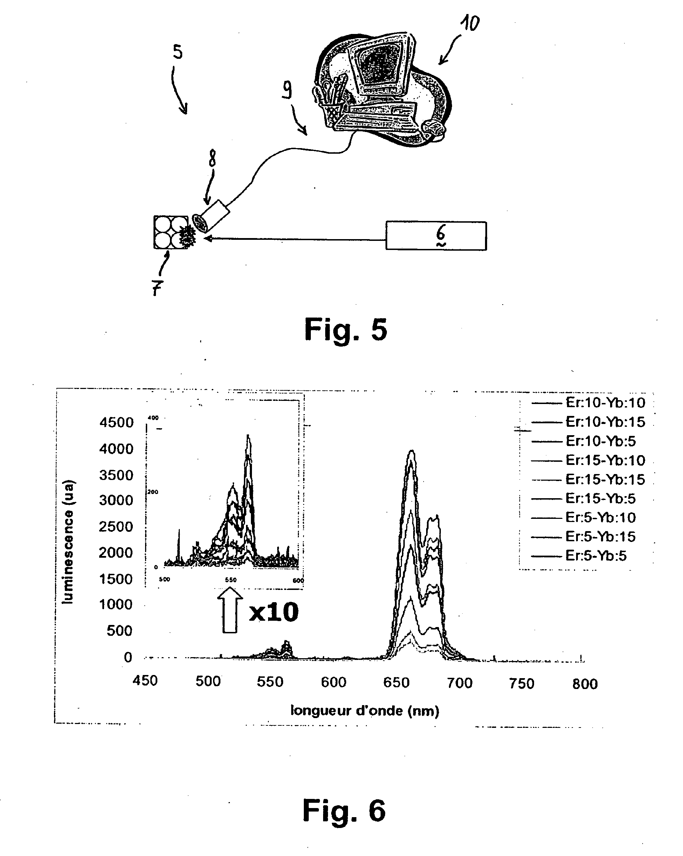

On a cherché à mettre en évidence, via le banc 5 de caractérisation optique illustré à la figure 5, les propriétés d'émission de nanocristaux d'oxyde de type convertisseurs élévateurs de fréquence qui ont été utilisés dans la méthode selon l'invention et qui répondent à la formule $Y_2O_3$:$Er^{3+}$, $Yb^{3+}$ (ces nanocristaux testés sont commercialisés par la société DGTec). On a étudié l'influence de différents taux de dopage en erbium (Er) et en ytterbium (Yb), ces taux variant chacun entre 5 % et 15%, et l'on a testé ces nanocristaux tant à l'état de poudre qu'en solution dans un liquide mimant les propriétés optiques des tissus.

Ce banc 5 comporte essentiellement :

- une source d'éclairage 6 laser, adaptée pour émettre des rayonnements dans le proche infrarouge à des longueurs d'onde d'excitation $\lambda_1$ et $\lambda_2$ respectivement égales à 980 nm et 815 nm,
- un échantillon 7 des nanocristaux d'oxyde $Y_2O_3$:$Er^{3+}$, $Yb^{3+}$,
- une fibre 8 avec filtre « XF 3100 » apte à recueillir l'émission de fluorescence de cet échantillon 7, et
- un spectromètre 9 à fibre relié à des moyens d'acquisition et d'affichage 10 des signaux obtenus.

On a placé ces nanocristaux d'oxyde en poudre, soit directement dans une cuve de spectrophotométrie de 1 cm de côté, soit dans un capillaire de 1 mm de diamètre placé dans une cuve remplie d'intra-lipide mimant les propriétés optiques des tissus.

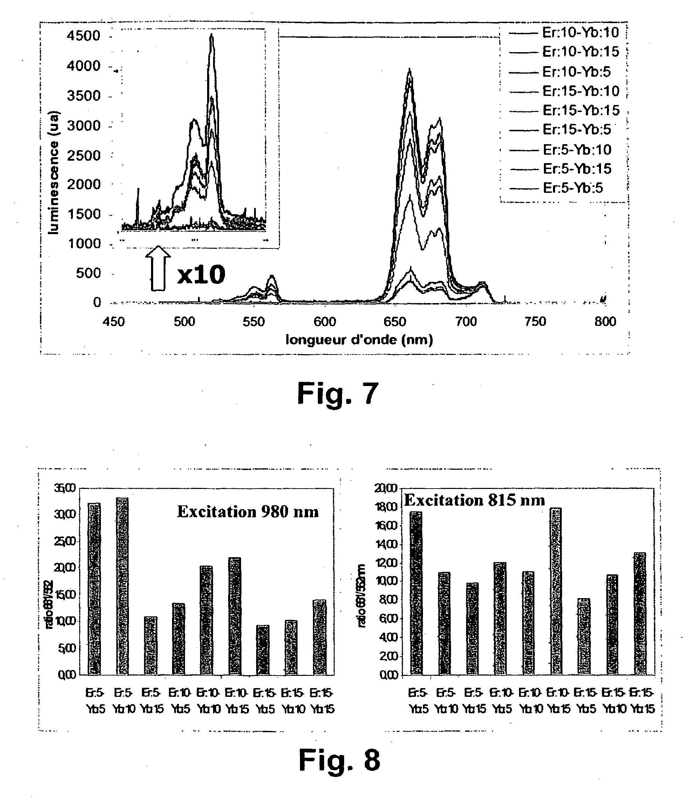

Lors de l'excitation séparée des nanocristaux $Y_2O_3$:$Er^{3+}$,$Yb^{3+}$ à l'état de poudre aux longueurs d'onde $\lambda_1$ = 980 nm et $\lambda_2$ = 815 nm, on a observé deux bandes d'émission $B_1$ et $B_2$, i.e. une émission verte à $\lambda'_1$ = 560 nm et une émission rouge plus intense à $\lambda'_2$ = 661 nm, comme illustré respectivement aux figures 6 et 7 qui se réfèrent respectivement à deux excitations à $\lambda_1$ et $\lambda_2$.

Ces différents taux de dopage en $Er^{3+}$ et en $Yb^{3+}$ utilisés en combinaison conduisent à des fluorophores ayant différents rapports d'émission entre la bande à $\lambda'_2$ = 661 nm et celle à $\lambda'_1$ = 560 nm, comme cela est illustré aux deux graphiques à barres de la figure 8 qui se réfèrent également à ces mêmes nanocristaux analysés à l'état de poudre (les mêmes taux combinés de dopage que ceux présentés aux figures 6 et 7 ont été repris en abscisse à la figure 8). Ces rapports d'émission diffèrent également suivant les longueurs d'onde d'excitation $\lambda_1$ et $\lambda_2$.

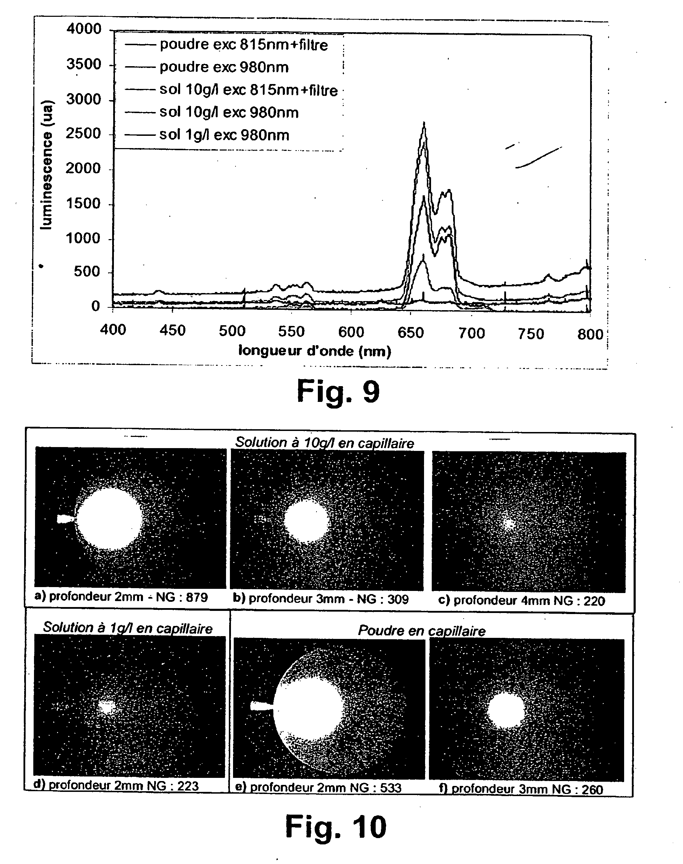

Bien que moins intenses qu'à l'état de poudre, on a vérifié que ces propriétés d'émission des nanocristaux $Y_2O_3$:$Er^{3+}$,$Yb^{3+}$, dont les taux de dopage sont de 10 % en $Er^{3+}$ et de 5 % en $Yb^{3+}$, sont conservées dans un milieu liquide de propriétés optiques similaires à celles des tissus biologiques, comme le montre la Figure 9.

Ce milieu liquide est constitué d'une solution d'intra-lipide formée de 80 % d'un mélange eau-encre et de 20 % d'intra-lipide, pour obtenir un coefficient d'absorption $\mu_a$ = 0,05 cm$^{-1}$ et un coefficient réduit de diffusion de $\mu'_s$ = 12 cm$^{-1}$, représentatif d'un tissu biologique.

On a rempli un capillaire de 1 mm de diamètre de ces nanocristaux, soit à l'état de poudre, soit en solution dans l'eau à différentes concentrations (10 g/l et 1 g/l), et l'on a placé ce capillaire au centre de la cuve de spectrophotométrie de 1 cm de côté ayant été remplie d'intra-lipide. En outre, on a utilisé un filtre « XF 3100 » pour deux essais d'excitation en poudre et en solution effectués à 815 nm, afin d'éliminer la diffusion.

La figure 9 montre qu'il y a bien émission de fluorescence à 550 nm et 660 nm, soit sensiblement aux longueurs d'onde $\lambda'_1$ et $\lambda'_2$ lors de l'excitation des nanocristaux à $\lambda_1$ = 980 nm et $\lambda_2$ = 815 nm.

En conclusion, ces fluorophores constitués de nanocristaux inorganiques semi-conducteurs de type convertisseur élévateur de fréquence satisfont bien, d'une part, à la condition de double excitation séquentielle à des longueurs d'ondes $\lambda_1$ et $\lambda_2$ séparées de plus de 100 nm ($\lambda_1$-$\lambda_2$ = 165 nm) et, d'autre part, à la condition d'émissions simultanées de fluorescence sensiblement identiques pour chaque excitation à $\lambda_1$ et $\lambda_2$, conditions requises pour la mise en oeuvre de la méthode de l'invention.

On notera que cette double excitation séquentielle permet de s'affranchir complètement des problèmes d'auto-fluorescence des tissus, notamment grâce aux propriétés d'élévation de fréquence des nanocristaux d'oxydes.

On notera également que les deux raies observées à 560 nm et 661 nm sont très stables en position et que leur intensité relative ne dépend que de la proportion des constituants de base. De plus, quel que soit l'environnement, aucun décalage spectral n'a été observé.

L'intervalle de longueur d'onde entre les deux émissions (d'environ 100 nm) est suffisant pour discriminer un fluorophore en profondeur d'un fluorophore en surface. En effet, un fluorophore en surface verra la contribution de la raie à 560 nm autant que celle à 661 nm et, *a contrario,* un fluorophore en profondeur verra la contribution de la raie à 560 nm nettement amoindrie par rapport à celle à 661 nm.

L'utilisation de ces nanocristaux inorganiques convertisseurs élévateurs de fréquence présente de plus les avantages suivants :

- on peut choisir les longueurs d'onde d'excitation dans une large plage, par exemple en fonction des tissus et/ou de la source d'éclairage: en effet, les nanocristaux $Y_2O_3$:10 % $Er^{3+}$, 5% $Yb^{3+}$ peuvent être excités avec deux longueurs d'onde (815 nm et 980 nm) correspondant à une très bonne transmission des photons dans les tissus, et pour lesquelles des lasers à bas prix sont commercialement disponibles ;
- on élimine ou on réduit sensiblement l'auto-fluorescence des tissus, grâce aux propriétés élévatrices de fréquence de ces nanocristaux ;
- on limite les problèmes de diffusion de la lumière d'excitation et de filtrage, grâce à des différences significatives entre longueurs d'onde d'excitation et d'émission (qui évitent le chevauchement entre ces dernières), des raies d'absorption/d'émission très fines, et des propriétés élévatrices de fréquence des nanocristaux d'oxydes selon l'invention ;
- ces nanocristaux élévateurs de fréquence peuvent être fabriqués à des tailles nanométriques et ne sont *a priori* pas toxiques, car très inertes chimiquement dans les conditions physiologiques ;
- ces nanocristaux présentent une grande stabilité en position des raies d'émission et d'excitation, facilitant ainsi le filtrage optique ; et
- ces nanocristaux peuvent être aisément fonctionnalisés, en vue de cibler des tissus ou organes biologiques.

Concernant ce dernier point, différents procédés de fonctionnalisation ont été décrits pour ces nanocristaux dans le but d'introduire des ligands biologiques, par exemple susceptibles de reconnaître des récepteurs cellulaires surexprimés à la surface de cellules tumorales, et l'on notera que ces procédés sont utilisables dans le cadre de l'invention.

### 3) Exemple d'application de la méthode selon l'invention :

On a utilisé la méthode d'imagerie optique selon l'invention dans un petit animal, tel qu'une souris, pour la détection de structures d'intérêt (tumeurs, organes). A cet effet, on a procédé à la détection de marqueurs à base de divers échantillons des nanocristaux inorganiques précités de formule $Y_2O_3$:10 % $Er^{3+}$, 5% $Yb^{3+}$ à travers un liquide mimant les propriétés optiques des tissus biologiques de cet animal.

On a placé un échantillon de ces nanocristaux dans un capillaire fermé (de 1,5 mm de diamètre), et on l'a introduit dans un fantôme simulant un milieu biologique connu ($\mu_a$ = 0,2 cm$^{-1}$ et $\mu'_s$ = 10 cm$^{-1}$). On a éclairé par en dessous le capillaire placé à différentes épaisseurs du fantôme, avec un laser à 980 nm (30 mW) selon le schéma précité de tomographie illustré à la figure 2. On a récupéré l'émission de ces nanocristaux grâce à une caméra placée au-dessus du fantôme.

Plus précisément, on a réalisé des essais avec ces nanocristaux en solution à 1 g/l (cliché d), 10 g/l (clichés a, b et c) et en poudre (clichés e et f).

La figure 10 présente les images d'émission de fluorescence et les niveaux de gris (NG) obtenus pour différentes concentrations de ces nanocristaux.

Cette figure 10 montre que ces nanocristaux de formule $Y_2O_3$:10 % Er, 5 % Yb en solution à 10 g/l dans un capillaire sont facilement détectables dans le fantôme jusqu'à 4 mm de profondeur, cette profondeur de pénétration permettant avantageusement de visualiser tous les organes d'un petit animal tel qu'une souris. On peut remarquer par ailleurs la diffusion importante qui existe à ces longueurs d'onde dans un milieu mimant les propriétés optiques (diffusion, absorption) des tissus.

### Revendications

1. Procédé d'imagerie optique d'au moins un tissu biologique dans lequel au moins un marqueur fluorescent a été introduit préalablement, comprenant les étapes suivantes :

   a) une excitation dudit ou de chaque marqueur par des rayonnements lumineux incidents et une détection de bandes d'émission relatives à des rayonnements fluorescents émis par ledit ou chaque marqueur en réponse à cette excitation, puis
   b) une analyse des intensités de fluorescence relatives auxdites bandes d'émission,
   **caractérisé en ce que** l'étape a) comprend :

   - une excitation séquentielle, par $\underline{n}$ longueurs d'onde d'excitation $\lambda_i$ incidentes différentes, dudit ou de chaque marqueur qui est apte à être excité par au moins deux de ces $\underline{n}$ longueurs d'ondes $\lambda_i$ et à émettre en réponse, pour chaque longueur d'onde $\lambda_i$, une série $S_i$ de $\underline{m}$ bandes d'émission $B_j$ simultanées présentant différentes longueurs d'onde maximales $\lambda'_j$ qui sont sensiblement les mêmes d'une série $S_i$ à une autre (où n et m sont indépendamment l'un de l'autre des entiers égaux ou supérieurs à 2, et où i et j varient respectivement de 1 à n et de 1 à m), et
   - une détection de ces séries $S_i$ au moins au nombre de deux qui comprennent chacune ces $\underline{m}$ bandes $B_j$ émises simultanément, pour en déduire à l'étape b) une estimation de la localisation tridimensionnelle dudit ou de chaque marqueur dans le ou chaque tissu et/ou des coefficients d'absorption moyens du ou de chaque tissu vis-à-vis des longueurs d'onde d'excitation $\lambda_i$.

2. Procédé d'imagerie selon la revendication 1, **caractérisé en ce que** ledit ou chaque marqueur est à base d'un fluorophore ou groupe de fluorophores qui est apte à être excité par ces longueurs d'ondes $\lambda_i$ et à émettre simultanément, en réponse à chacune d'entre elles, ces $\underline{m}$ bandes $B_j$.

3. Procédé d'imagerie selon la revendication 1 ou 2, **caractérisé en ce que** l'étape b) comprend également une détermination d'un ou de plusieurs ratio(s) d'émission entre les $\underline{m}$ longueurs d'onde maximales $\lambda'_j$ et d'un ou plusieurs ratio(s) de transmission du ou de chaque tissu entre les $\underline{n}$ longueurs d'onde d'excitation $\lambda_i$, pour l'obtention d'une cartographie d'émission du ou de chaque tissu.

4. Procédé d'imagerie optique selon une des revendications 1 à 3, **caractérisé en ce que** l'on utilise un dispositif d'imagerie optique de type fonctionnant en transmission, comportant une source (1) pour lesdits rayonnements incidents et un détecteur (2) qui sont situés respectivement des deux côtés dudit ou de chaque tissu à imager.

5. Procédé d'imagerie optique selon une des revendications 1 à 3, **caractérisé en ce que** l'on utilise un dispositif d'imagerie optique comportant une source pour lesdits rayonnements incidents et un détecteur qui sont situés tous deux d'un même côté dudit ou chaque tissu à imager.

6. Procédé d'imagerie optique selon la revendication 5, **caractérisé en ce que** l'on déduit également de l'étape a) une cartographie de rétrodiffusion dudit ou de chaque tissu aux $\underline{n}$ longueurs d'onde d'excitation $\lambda_i$.

7. Procédé d'imagerie optique selon une des revendications précédentes, **caractérisé en ce que** les $\underline{n}$ longueurs d'onde d'excitation $\lambda_i$ sont décalées deux à deux entre elles d'un intervalle au moins égal à 100 nm.

8. Procédé d'imagerie optique selon une des revendications précédentes, **caractérisé en ce que** les $\underline{m}$ longueurs d'onde maximales $\lambda'_j$ desdites bandes d'émission $B_j$ sont décalées deux à deux entre elles d'un intervalle au moins égal à 100 nm.

9. Procédé d'imagerie selon une des revendications précédentes, **caractérisé en ce que** les $\underline{n}$ longueurs d'onde d'excitation $\lambda_i$ sont toutes comprises entre 750 nm et 1000 nm.

10. Procédé d'imagerie selon la revendication 9, **caractérisé en ce que** les $\underline{m}$ longueurs d'onde maximales $\lambda'_j$ desdites bandes d'émission $B_j$ sont toutes comprises entre 450 nm et 800 nm.

11. Procédé d'imagerie optique selon une des revendications précédentes, **caractérisé en ce que** l'étape a) comprend :

   - l'excitation successive, par deux longueurs d'ondes d'excitation différentes $\lambda_1$ et $\lambda_2$, dudit marqueur qui est apte à être excité par ces deux longueurs d'ondes $\lambda_1$ et $\lambda_2$ et à émettre en réponse sensiblement une même série $S_1$, $S_2$ de deux bandes d'émission $B_1$ et $B_2$ présentant respectivement deux longueurs d'onde maximales différentes $\lambda'_1$ et $\lambda'_2$ ($\underline{n} = \underline{m} = 2$), et
   - la détection de ces deux séries $S_1$ et $S_2$ comprenant chacune de ces deux bandes $B_1$ et $B_2$ émises simultanément.

12. Procédé d'imagerie selon une des revendications précédentes, **caractérisé en ce que** ledit ou chaque marqueur comprend un fluorophore à base d'au moins un nanocristal inorganique semi-conducteur de type convertisseur élévateur de fréquence.

13. Procédé d'imagerie selon la revendication 12, **caractérisé en ce que** ledit ou chaque nanocristal comprend au moins :

   - un oxyde ou un oxysulfure d'un métal choisi dans le groupe constitué par l'yttrium, le vanadium et les terres rares, et
   - un ion émetteur, tel qu'un cation de terre rare.

14. Procédé d'imagerie selon une des revendications précédentes, **caractérisé en ce que** ledit ou chaque marqueur comprend en outre au moins un élément, par exemple choisi dans le groupe constitué par les chélates de gadolinium, les nanoparticules d'oxydes de fer et les nanoparticules de gadolinium, qui est apte à faire dudit marqueur un agent de contraste présentant une autre propriété utilisable par exemple dans l'imagerie par résonance magnétique, la tomographie d'émission de positons, la gammatomographie ou l'imagerie aux rayons X.

15. Procédé d'imagerie selon la revendication 13 ou 14, **caractérisé en ce que** ledit ou chaque nanocristal est à base d'un oxyde d'yttrium répondant à la formule $Y_2O_3$ : $Er^{3+}$, $Yb^{3+}$, où Er et Yb sont respectivement l'erbium et l'ytterbium et sont chacun présents dans ledit ou chaque nanocristal selon un taux de dopage allant de 1 % à 20 %.

16. Procédé d'imagerie selon une des revendications précédentes, **caractérisé en ce que** ledit ou chaque tissu est de type *in vivo*, ledit ou chaque marqueur étant à base d'un conjugué fluorophore / ligand biologique, tel qu'un conjugué nanocristal inorganique semi-conducteur fonctionnalisé de type convertisseur élévateur de fréquence / biomolécule.

17. Procédé d'imagerie selon une des revendications précédentes, **caractérisé en ce que** l'on utilise une cartographie d'émission et/ou d'absorption et/ou de rétrodiffusion spécifiquement obtenue(s) à l'étape c) pour ledit ou chaque tissu, pour délimiter au moins une région d'intérêt dudit ou chaque tissu à analyser par tomographie.

18. Procédé d'imagerie selon la revendication 17, **caractérisé en ce que** l'on utilise cette délimitation de ladite ou chaque région d'intérêt propre audit ou à chaque tissu comme point de départ pour une reconstruction d'images par tomographie.

19. Procédé d'imagerie selon la revendication 17 ou 18, **caractérisé en ce que** ladite ou lesdites cartographie(s) sont relatives à un corps entier d'animal (4).

**Claims**

1. A method of optically imaging at least one biological tissue, in which at least one fluorescent marker has been beforehand introduced, the method comprising the following steps:

   a) exciting said at least one marker by incident light radiations and detecting bands of emission relating to fluorescence emitted by said at least one marker in response to that excitation; then
   b) analyzing the intensities of fluorescence relative to said emission bands;

   **characterized in that** the step a) comprises:

   · sequentially exciting said at least one marker at $\underline{n}$ different incident excitation wavelengths $\lambda_i$, said at least one marker being adapted to be excited by at least two of these $\underline{n}$ wavelengths $\lambda_i$ and to emit in response to each wavelength $\lambda_i$ a series $S_i$ of $\underline{m}$ simultaneous emission bands $B_j$ having different maximum wavelengths $\lambda'_j$ that are substantially the same from one series $S_i$ to another (where $\underline{n}$ and $\underline{m}$ are independent integers equal to or greater than 2 and where i and $\underline{j}$ respectively vary from 1 to $\underline{n}$ and from 1 to $\underline{m}$); and
   · detecting at least two of these series $S_i$ that each comprise these $\underline{m}$ bands $B_j$ emitted simultaneously in order to deduce therefrom in the step b) an estimate of the three-dimensional location of said marker in the tissue(s) and/or the mean absorption coefficients of the tissue(s) for the excitation wavelengths $\lambda_i$.

2. An imaging method according to claim 1, **characterized in that** said marker is based on a fluorophore or a group of fluorophores that is adapted to be excited by these wavelengths $\lambda_i$ and to emit these $\underline{m}$ bands $B_j$ simultaneously in response to each of them.

3. An imaging method according to claim 1 or 2, **characterized in that** the step b) also comprises determining one or more emission ratio(s) between the $\underline{m}$ maximum wavelengths $\lambda'_j$ and one or more transmission ratio(s) of said at least one tissue between the $\underline{n}$ excitation wavelengths $\lambda_i$, to obtain an emission map of said at least one tissue.

4. An optical imaging method according to any of claims 1 to 3, **characterized in that** the method uses an optical imaging device of the type operating in transmission mode and including a source of said incident radiations and a detector which are situated on respective both opposite sides of said at least one tissue to be imaged.

5. An optical imaging method according to any of claims 1 to 3, **characterized in that** the method uses an optical imaging device including a source of said incident radiations and a detector both of which are situated on the same side of said at least one tissue to be imaged.

6. An optical imaging method according to claim 5, **characterized in that** there is also deduced in the step a) a backscattering map of the tissue at the $\underline{n}$ excitation wavelengths $\lambda_i$.

7. An optical imaging method according to any of the preceding claims, **characterized in that** the $\underline{n}$ excitation wavelengths $\lambda_i$ are offset in pairs by an interval of at least 100 nm.

8. An optical imaging method according to any of the preceding claims, **characterized in that** the $\underline{m}$ maximum wavelengths $\lambda'_j$ of said emission bands $B_j$ are offset in pairs by an interval of at least 100 nm.

9. An imaging method according to any of the preceding claims, **characterized in that** the $\underline{n}$ excitation wavelengths $\lambda_i$ are all of between 750 nm and 1000 nm.

**10.** An imaging method according to claim 9, **characterized in that** the $\underline{m}$ maximum wavelengths $\lambda'_j$ of said emission bands $B_j$ are all of between 450 nm and 800 nm.

**11.** An optical imaging method according to any of the preceding claims, **characterized in that** the step a) comprises:

· successively exciting said at least one marker at two different excitation wavelengths $\lambda_1$ and $\lambda_2$, said marker being adapted to be excited by these two wavelengths $\lambda_1$ and $\lambda_2$ and to emit in response substantially the same series $S_1$, $S_2$ of two emission bands $B_1$ and $B_2$ having respective different maximum wavelengths $\lambda'_1$ and $\lambda'_2$ ($\underline{n} = \underline{m} = 2$); and

· detecting the two series $S_1$ and $S_2$ each comprising the two bands $B_1$ and $B_2$ emitted simultaneously.

**12.** An imaging method according to any of the preceding claims, **characterized in that** the marker comprises a fluorophore based on at least one up-converting semiconductor inorganic nanocrystal.

**13.** An imaging method according to claim 12, **characterized in that** said nanocrystal includes at least:

· an oxide or an oxysulfide of a metal chosen from the group consisting of yttrium, vanadium and the rare earths; and

· an emitter ion, such as a rare earth cation.

**14.** An imaging method according to any of the preceding claims, **characterized in that** said at least one marker further comprises an element chosen from the group consisting of chelates of gadolinium, nanoparticles of oxides of iron and nanoparticles of gadolinium, which element is adapted to make said marker act as a contrast agent having another property usable for example in magnetic resonance imaging, positron emission tomography, gammatomography or X-ray imaging.

**15.** An imaging method according to claim 13 or 14, **characterized in that** said nanocrystal is based on an yttrium oxide having the formula $Y_2O_3 : Er^{3+}, Yb^{3+}$, where Er and Yb are respectively erbium and ytterbium and each is present in said nanocrystal at a doping rate from 1% to 20%.

**16.** An imaging method according to any of the preceding claims, **characterized in that** said at least one tissue is of the *in vivo* type, said at least one marker being based on a fluophore/biological ligand conjugate such as an up-converting semiconductor nanocrystal/biomolecule conjugate.

**17.** An imaging method according to any of the preceding claims, **characterized in that** an emission and/or absorption and/or backscattering map specifically obtained in the step c) is used to define at least one region of interest of said or each tissue to be analyzed by tomography.

**18.** An imaging method according to claim 17, **characterized in that** said definition of said at least one region of interest specific to said or each tissue is used as a starting point for image reconstruction by tomography.

**19.** An imaging method according to claim 17 or 18, **characterized in that** said map(s) relate to an entire animal body.

**Patentansprüche**

**1.** Optisches Abbildungsverfahren von mindestens einem biologischen Gewebe, in welches zuvor mindestens ein fluoreszierender Marker eingebracht worden ist, umfassend die folgenden Schritte:

a) Eine Anregung von diesem oder einem beliebigen Marker durch auftreffende Lichtstrahlung und eine Detektion von Emissionsbanden betreffend die emittierte fluoreszierende Strahlung durch diesen oder einen beliebigen Marker in Antwort auf diese Anregung, danach

b) eine Analyse der Fluoreszenzintensitäten bezogen auf besagte Emissionsbanden,

**dadurch gekennzeichnet, dass** Schritt a) umfasst:

- eine sequentielle Anregung mit $\underline{n}$ verschiedenen auftreffenden Excitationswellenlängen $\lambda_i$ von diesem oder einem beliebigen Marker, der in der Lage ist, durch mindestens zwei der $\underline{n}$ Wellenlängen $\lambda_i$ angeregt zu werden,

und als Antwort auf jede Wellenlänge $\lambda_i$ eine Serie $S_i$ an $\underline{m}$ Emissionsbanden $B_j$ zu emittieren, die gleichzeitig verschiedene maximale Wellenlängen $\lambda'_j$ aufweisen, die im Wesentlichen die selben für eine Serie $S_i$ oder eine andere sind (wo n und m unabhängig voneinander ganze Zahlen gleich oder größer als 2 sind, und wobei i und j von 1 bis n, beziehungsweise von 1 bis m variieren, und

- eine Detektion dieser Serien $S_i$ in mindestens einer Anzahl von zwei, wobei jede diese gleichzeitig emittierten $\underline{m}$ Banden $B_j$ umfassen, um daraus in Schritt b) eine Einschätzung der dreidimensionalen Lokalisation von diesem oder einem beliebigen Marker in diesem oder einem beliebigen Gewebe und/oder der durchschnittlichen Absorptionskoeffizienten des oder eines beliebigen Gewebes gegenüber den Excitationswellenlängen $\lambda_i$ ab-zuleiten.

2. Abbildungsverfahren nach Anspruch 1, **dadurch gekennzeichnet, dass** dieser oder ein beliebiger Marker aus einem Fluorophor oder einer Gruppe von Fluorophoren besteht, der/die in der Lage ist, durch diese Wellenlängen $\lambda_i$ angeregt zu werden und gleichzeitig als Antwort auf jede von ihnen die $\underline{m}$ Banden $B_j$ zu emittieren.

3. Abbildungsverfahren nach Anspruch 1 oder 2, **dadurch gekennzeichnet, dass** Schritt b) ebenfalls eine Bestimmung eines oder mehrerer Emissionsverhältnisse(s) zwischen den maximalen m Wellenlängen $\lambda'_j$ und eines oder mehrerer Verhältnisse(s) der Übertragung des oder eines beliebigen Gewebes zwischen den $\underline{n}$ Excitationswellenlängen $\lambda_i$ umfasst, zur Erzeugung einer Emissionskartographie des oder eines beliebigen Gewebes.

4. Optisches Abbildungsverfahren nach einem der Ansprüche 1 bis 3, **dadurch gekennzeichnet, dass** man eine optische Abbildungsvorrichtung, die über Transmission funktioniert, verwendet, die eine Quelle (1) für die auftreffende Strahlung und einen Detektor (2) beinhaltet, die sich jeweils an beiden Seiten von diesem oder einem beliebigen abzubildenden Gewebe befinden.

5. Optisches Abbildungsverfahren nach einem der Ansprüche 1 bis 3, **dadurch gekennzeichnet, dass** man eine optische Abbildungsvorrichtung verwendet, die eine Quelle für die auftreffende Strahlung und einen Detektor beinhaltet, die sich beide auf der selben Seite von diesem oder einem beliebigen abzubildenden Gewebe befinden.

6. Optisches Abbildungsverfahren nach Anspruch 5, **dadurch gekennzeichnet, dass** man aus Schritt a) ebenfalls eine Rückstreuungskartographie von diesem oder einem beliebigen Gewebe mit $\underline{n}$ Excitationswellenlängen $\lambda_i$ ab-leitet.

7. Optisches Abbildungsverfahren nach einem der vorhergehenden Ansprüche, **dadurch gekennzeichnet, dass** die $\underline{n}$ Excitationswellenlängen $\lambda_i$ paarweise untereinander in einem Intervall von mindestens 100 nm versetzt sind.

8. Optisches Abbildungsverfahren nach einem der vorhergehenden Ansprüche, **dadurch gekennzeichnet, dass** die $\underline{m}$ maximalen Wellenlängen $\lambda'_j$ der Emissionsbanden $B_j$ paarweise untereinander in einem Intervall von mindestens 100 nm versetzt sind.

9. Optisches Abbildungsverfahren nach einem der vorhergehenden Ansprüche, **dadurch gekennzeichnet, dass** die $\underline{n}$ Excitationswellenlängen $\lambda_i$ alle zwischen 750 nm und 1000 nm liegen.

10. Optisches Abbildungsverfahren nach Anspruch 9, **dadurch gekennzeichnet, dass** die $\underline{m}$ maximalen Wellenlängen $\lambda'_j$ dieser Emissionsbanden $B_j$ alle zwischen 450 nm und 800 nm liegen.

11. Optisches Abbildungsverfahren nach einem der vorhergehenden Ansprüche, **dadurch gekennzeichnet, dass** Schritt a) umfasst:

- die sukzessive Anregung durch zwei verschiedene Excitationswellenlängen $\lambda_1$ und $\lambda_2$, des Markers, der in der Lage ist, durch die zwei Wellenlängen $\lambda_1$ und $\lambda_2$ angeregt zu werden und in Antwort darauf eine im We-sentlichen gleiche Serie $S_1$, $S_2$ von zwei Emissionsbanden $B_1$ und $B_2$ zu emittieren, die jeweils zwei unter-schiedliche maximale Wellenlängen $\lambda'_1$ und $\lambda'_2$ ($\underline{n} = \underline{m} = 2$) aufweisen, und
- die Detektion der zwei Serien $S_1$ und $S_2$, die jede der zwei gleichzeitig emittierten Banden B 1 und B2 umfassen.

12. Abbildungsverfahren nach einem der vorhergehenden Ansprüche, **dadurch gekennzeichnet, dass** dieser oder ein beliebiger Marker einen Fluorophor aus mindestens einem anorganischen Nanokristall-Halbleiter der Art eines Frequenz-Aufwärtswandlers umfasst.

**13.** Abbildungsverfahren nach Anspruch 12, **dadurch gekennzeichnet, dass** der oder ein beliebiger Nanokristall mindestens umfasst:

- ein Oxid oder ein Sulfoxid eines Metalls ausgewählt aus der Gruppe bestehend aus Yttrium, Vanadium und den seltenen Erden, und
- einen Ionengeber, wie ein Kation der seltenen Erde.

**14.** Abbildungsverfahren nach einem der vorhergehenden Ansprüche, **dadurch gekennzeichnet, dass** dieser oder ein beliebiger Marker außerdem mindestens ein Element, zum Beispiel ausgewählt aus der Gruppe bestehend aus den Gadolinium-Chelaten, den Eisenoxid-Nanopartikeln, und den Gadolinium-Nanopartikeln umfasst, das in der Lage ist, aus dem Marker ein Kontrastmittel zu machen, das eine andere Eigenschaft aufweist, die nützlich ist zum Beispiel bei der Abbildung mittels Magnetresonanz, der Positronenemissionstomographie, der Gammatomographie oder der Röntgenstrahlungabbildung.

**15.** Abbildungsverfahren nach Anspruch 13 oder 14, **dadurch gekennzeichnet, dass** der oder ein beliebiger Nanokristall aus Yttriumoxid besteht gemäß der Formel $Y_2O_3$: $Er^{3+}$, $Yb^{3+}$, wobei Er und Yb jeweils Erbium und Ytterbium sind, und in diesem oder einem beliebigen Nanokristall nach einer Dopingrate 1% bis 20% vorliegen.

**16.** Abbildungsverfahren nach einem der vorhergehenden Ansprüche, **dadurch gekennzeichnet dass** dieses oder ein beliebiges Gewebe der Art *in vivo* ist, dieser oder ein beliebiger Marker aus einer Konjugation Fluorophor/ biologischer Ligand besteht, wie beispielsweise eine funktionalisierte anorganische Nanokristall-Halbleiter-Konjugation der Art Frequenz-Aufwärtswandler/Biomolekül.

**17.** Abbildungsverfahren nach einem der vorhergehenden Ansprüche, **dadurch gekennzeichnet, dass** man eine Emissions- und/oder Absorptions- und/oder Rückstreuungskartographie, die spezifisch in Schritt c) für das oder ein beliebiges Gewebe erzeugt wurde(n), verwendet, zum Eingrenzen von mindestens einer Region von Interesse dieses oder eines beliebigen mittels Tomographie zu analysierenden Gewebes.

**18.** Abbildungsverfahren nach Anspruch 17, **dadurch gekennzeichnet, dass** man diese Eingrenzung dieser oder einer beliebigen Region von Interesse bezüglich dieses oder eines beliebigen Gewebes als Ausgangspunkt für eine Bilderrekonstruktion mittels Tomographie verwendet.

**19.** Abbildungsverfahren nach Anspruch 17 oder 18, **dadurch gekennzeichnet, dass** die Kartographie(n) auf einen gesamten Tierkörper (4) bezogen sind.

$\lambda_1$ •————————→• $\lambda'_1$

$\lambda_2$ •————————→• $\lambda'_2$

Excitation                    Emission

# Fig. 1

$\underset{\sim}{2}$

$\underset{\sim}{3}$

4

L

x

y

z

1

# Fig. 2

# Fig. 3

# Fig. 4

Fig. 5

Fig. 6

EP 1 849 413 B1

Fig. 7

Fig. 8

19

**Fig. 9**

**Fig. 10**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5370119 A

- FR 2812662 A